(19) **Europäisches Patentamt / European Patent Office / Office européen des brevets**

(11) **EP 3 427 654 A1**

(12) **EUROPEAN PATENT APPLICATION**
published in accordance with Art. 153(4) EPC

(43) Date of publication:
**16.01.2019 Bulletin 2019/03**

(21) Application number: **17782516.3**

(22) Date of filing: **14.04.2017**

(51) Int Cl.:
**A61B 5/02** *(2006.01)*      **A61B 5/022** *(2006.01)*
**A61B 5/029** *(2006.01)*      **A61B 5/08** *(2006.01)*

(86) International application number:
**PCT/JP2017/015284**

(87) International publication number:
**WO 2017/179703 (19.10.2017 Gazette 2017/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **15.04.2016 JP 2016082463**

(71) Applicants:
- **Omron Corporation**
  **Kyoto-shi, Kyoto 600-8530 (JP)**
- **Omron Healthcare Co., Ltd.**
  **Muko-shi, Kyoto 617-0002 (JP)**

(72) Inventors:
- **NAKAJIMA, Hiroshi**
  **Kyoto-shi**
  **Kyoto 600-8530 (JP)**
- **WADA, Hirotaka**
  **Kyoto-shi**
  **Kyoto 600-8530 (JP)**
- **TSUCHIYA, Naoki**
  **Kyoto-shi**
  **Kyoto 600-8530 (JP)**
- **KASAI, Masaaki**
  **Kyoto-shi**
  **Kyoto 600-8530 (JP)**
- **KAN, Eriko**
  **Kyoto-shi**
  **Kyoto 600-8530 (JP)**
- **UENOYAMA, Toru**
  **Tokyo 108-0075 (JP)**
- **OBAYASHI, Keiichi**
  **Kyoto-shi**
  **Kyoto 600-8530 (JP)**
- **KOKUBO, Ayako**
  **Kyoto-shi**
  **Kyoto 600-8530 (JP)**
- **OTA, Yuya**
  **Kyoto-shi**
  **Kyoto 600-8530 (JP)**
- **SHIGA, Toshikazu**
  **Muko-shi**
  **Kyoto 617-0002 (JP)**
- **KUWABARA, Mitsuo**
  **Muko-shi**
  **Kyoto 617-0002 (JP)**
- **SATO, Hironori**
  **Muko-shi**
  **Kyoto 617-0002 (JP)**
- **MIYAGAWA, Ken**
  **Muko-shi**
  **Kyoto 617-0002 (JP)**
- **TSUTSUMI, Masakazu**
  **Muko-shi**
  **Kyoto 617-0002 (JP)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(54) **BIOLOGICAL INFORMATION ANALYSIS DEVICE AND SYSTEM, AND PROGRAM**

(57)      A biological information analysis device includes: an indicator extraction unit configured to acquire, from time-series data regarding blood pressure waveforms consecutively measured by a sensor that is configured to be worn on a body part of a user and to be capable of non-invasively measuring a blood pressure waveform for each heartbeat, data regarding a blood pressure waveform in a pre-exercise period that is a period of time before the user performs exercise, and data regarding a blood pressure waveform in a post-exercise period that is a period of time after the user performs the exercise, and extract an indicator indicating the influence of the exercise on the user's cardiac function, based on characteristics of the blood pressure waveform in the

**(Cont. next page)**

pre-exercise period and characteristics of the blood pressure waveform in the post-exercise period; and a processing unit configured to perform processing that is based on the indicator thus extracted.

# FIG. 5

## Description

TECHNICAL FIELD

The present invention relates to technology for acquiring useful information from a blood pressure waveform that has been measured.

RELATED ART

There is a known technology for measuring changes in the internal pressure of a radial artery and recording the shape of a pressure pulse wave (blood pressure waveform). Patent Document 1 (JP 2008-61824A) discloses that a blood pressure waveform is measured using a tonometry method, and pieces of information such as an AI (Augmentation Index) value, a pulse wave period, a baseline fluctuation rate, sharpness, and an ET (Ejection Time) are acquired from the blood pressure waveform. Also, Patent Document 2 (JP 2005-532111A) discloses that a blood pressure waveform is measured using a wristwatch-type blood pressure meter, in which a mean arterial pressure, a mean systolic pressure, a mean diastolic pressure, a mean systolic pressure indicator, and a mean diastolic pressure indicator are calculated from the blood pressure waveform, and an alert is output when any of these values deviates from a reference value.

RELATED ART DOCUMENTS

PATENT DOCUMENTS

Patent Document 1: JP 2008-61824A
Patent Document 2: JP 2005-532111A

SUMMARY OF THE INVENTION

PROBLEM TO BE SOLVED BY THE INVENTION

It is known that exercise has the effect of improving blood pressure. However, it is necessary to continue exercise for a certain period of time to achieve the effect of lowering blood pressure. Therefore, there is a problem in that a user cannot imagine how much effect exercise has on the user, and the user cannot have high motivation to perform exercise. Also, too much exercise can actually be harmful to the user's body. However, the user does not know how much load is placed on his/her heart and so on by the exercise that he/she is performing, and therefore there is a problem in which the user cannot determine what exercise is risky for the user, or how much exercise is appropriate for the user.

The inventors of the present invention have been worked hard to develop a blood pressure measurement device that can accurately measure an ambulatory blood pressure waveform for each heartbeat, and to put such a device into practical use. Through experiments performed on subjects during the development phase, the inventors have found that various kinds of useful information can be extracted from data regarding ambulatory blood pressure waveforms that have been consecutively measured.

Therefore, the present invention aims to provide a novel technology for evaluating the degree of influence of exercise on cardiac function, based on data regarding blood pressure waveforms.

MEANS FOR SOLVING THE PROBLEMS

To achieve the above-described aim, the present invention employs the following configurations.

A biological information analysis device according to the present invention is an biological information analysis device including: an indicator extraction unit configured to acquire, from time-series data regarding blood pressure waveforms consecutively measured by a sensor that is configured to be worn on a body part of a user and to be capable of non-invasively measuring a blood pressure waveform for each heartbeat, data regarding a blood pressure waveform in a pre-exercise period that is a period of time before the user performs exercise, and data regarding a blood pressure waveform in a post-exercise period that is a period of time after the user performs the exercise, and extract an indicator indicating the influence of the exercise on the user's cardiac function, based on characteristics of the blood pressure waveform in the pre-exercise period and characteristics of the blood pressure waveform in the post-exercise period; and a processing unit configured to perform processing that is based on the indicator thus extracted.

Exercise causes a temporal change in a blood pressure waveform, and upon finishing the exercise, the blood pressure waveform gradually returns to its original state. The amount of change in the blood pressure waveform and the returning time and the returning speed of the blood pressure waveform in such a case depend on the content of the exercise that the user performed, and characteristics related to the user's cardiac function. Therefore, it is possible to quantitatively evaluate the influence of exercise on the user's cardiac function by using characteristics of the blood pressure waveform in the pre-exercise period and the blood pressure waveform in the post-exercise period as with the configuration according to the present invention.

Specifically, the indicator extraction unit may be configured to calculate the indicator based on a difference (the amount of change) between characteristics of the blood pressure waveform in the pre-exercise period and characteristics of the blood pressure waveform in the post-exercise period, or calculate the indicator based on an amount of time required for the characteristics of the blood pressure waveform, which has changed as a result of the exercise, to return to a state thereof in the pre-exercise period, or calculate the indicator based on a speed at which the characteristics of the blood pressure waveform, which has changed as a result of the exercise, return to a state thereof in the pre-exercise period. Also, these may be combined as appropriate. As described above, the amount of change in a blood pressure waveform and the returning time and the returning speed of a blood pressure waveform depend on the content of the exercise that has been performed and on characteristics related to the user's cardiac function. Therefore, it is possible to appropriately evaluate the influence of exercise on the user's cardiac function by at least focusing on the amount of change in a blood pressure waveform, the returning time, and the returning speed.

The indicator extraction unit may be configured to use, as characteristics of blood pressure waveforms, at least one of an AI (Augmentation Index), a systolic blood pressure, a diastolic blood pressure, a mean blood pressure, a heart rate, and a difference in time between a systolic blood pressure and a late systolic pressure. These characteristics may be used because they temporarily change (decrease or increase) as a result of exercise, and gradually return to their original states after the exercise.

It is preferable that the processing unit is configured to evaluate the degree of influence of the exercise on cardiac function, based on the indicator and an indicator that is related to the intensity and/or the duration of the exercise. Furthermore, it is preferable that the processing unit is configured to perform processing to output information regarding the degree of influence. By providing such information, it is possible to enable the user to grasp the exercise that he/she has performed and the influence of the exercise on the user's cardiac function in an objective and convincing manner.

Note that the present invention can be interpreted as a biological information analysis device or system that is provided with at least one of the above-described configurations or at least one of the above-described functions. The present invention can also be interpreted as a biological information analysis method that includes at least part of the above-described processing, or a program that causes a computer to execute such a method, or a computer-readable recording medium on which such a program is recorded in a non-transitory manner. The present invention can be formed by combining the above-described configurations and the above-described kinds of processing with each other unless no technical inconsistency occurs.

EFFECTS OF THE INVENTION

According to the present invention, it is possible to provide a novel technology for evaluating the degree of influence of exercise on cardiac function, based on data regarding blood pressure waveforms.

BRIEF DESCRIPTION OF THE DRAWINGS

FIG. 1 shows a schematic external configuration of a biological information analysis system 10.
FIG. 2 is a block diagram showing a hardware configuration of the biological information analysis system 10.
FIG. 3 is a cross-sectional view schematically showing a configuration of a blood pressure measurement unit 20 and a state in which measurement is performed.
FIG. 4 shows a blood pressure waveform that is measured by the blood pressure measurement unit 20.
FIG. 5 is a block diagram illustrating processing that is performed by a biological information analysis device 1.
FIG. 6 shows a waveform (a blood pressure waveform) of a pressure pulse wave from a radial artery corresponding to one heartbeat.
FIG. 7 is a flowchart for processing that is performed to evaluate an influence of exercise on cardiac function according to Example 1.
FIG. 8 is a diagram showing changes in blood pressure waveforms and a characteristic amount F of the blood pressure waveforms within a pre-exercise period, a target period (an exercise period), and a post-exercise period according to Example 1.
FIG. 9 shows an example of a chart for determining a result of evaluation according to Example 1.
FIG. 10 shows examples of pieces of evaluation result data according to Example 1.
FIG. 11 shows examples of indicators T and V in a case where AI values are used as values of the characteristic amount F of a blood pressure waveform according to Example 1.
FIG. 12 shows an example of an information output screen.
FIG. 13 shows an example of an information output screen.

EMBODIMENTS OF THE INVENTION

The following describes a preferred embodiment of the present invention with reference to the drawings. Note that the following descriptions of components may be modified as appropriate depending on the configuration of a device to which the present invention is applied, and on various conditions, and the scope of the present invention is not intended to be limited to the following descriptions.

Biological Information Analysis System

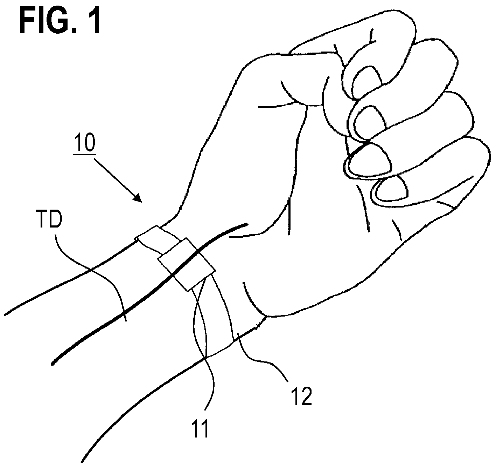

FIG. 1 shows a schematic external configuration of a biological information analysis system 10 according to an embodiment of the present invention. FIG. 1 shows a state in which the biological information analysis system 10 is worn on the left wrist. The biological information analysis system 10 includes a main body 11 and a belt 12 that is fixed to the main body 11. The biological information analysis system 10 is a so-called wearable device, and is worn such that the main body 11 is in contact with the skin on the palm side of the wrist, and the main body 11 is located over a radial artery TD that lies beneath the skin. Although the device is configured to be worn on the radial artery TD in the present embodiment, the device may be configured to be worn on another superficial artery.

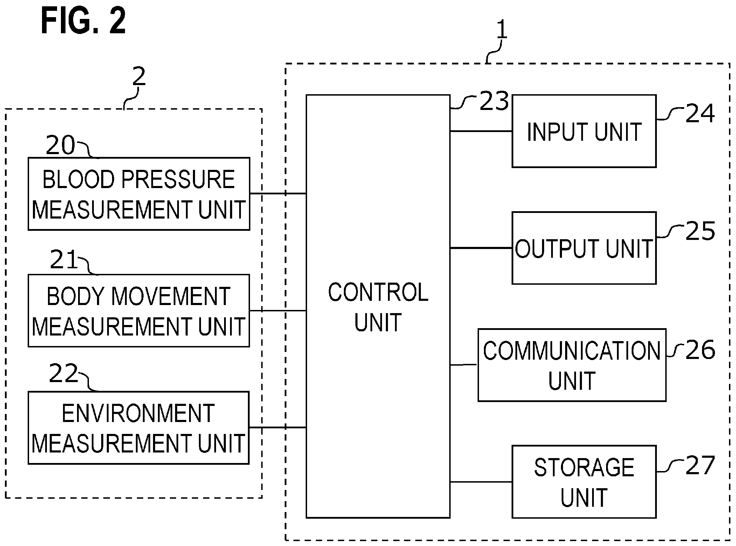

FIG. 2 is a block diagram showing a hardware configuration of the biological information analysis system 10. In general, the biological information analysis system 10 includes a measurement unit 2 and the biological information analysis device 1. The measurement unit 2 is a device that performs measurement to acquire information that is used to analyze biological information, and includes a blood pressure measurement unit 20, a body movement measurement unit 21, and an environment measurement unit 22. However, note that the configuration of the measurement unit 2 is not limited to that shown in FIG. 2. For example, a unit that measures biological information other than blood pressure or body movement (e.g. body temperature, blood-sugar level, or brain waves) may be added. Also, any unit that is not used in the example described below is not an essential component, and may be omitted from the biological information analysis system 10. The biological information analysis device 1 is a device that analyzes biological information based on information acquired from the measurement unit 2, and includes a control unit 23, an input unit 24, an output unit 25, a communication unit 26, and a storage unit 27. The units 20 to 27 are connected to each other so that signals can be exchanged between them via a local bus or other signal lines. The biological information analysis system 10 also includes a power supply (a battery), which is not shown.

The blood pressure measurement unit 20 measures a pressure pulse wave from the radial artery TD by using a tonometry method. The tonometry method is for forming a flat area in the artery TD by pressing the artery from the skin with appropriate pressure, adjusting the balance between the internal pressure and the external pressure of the artery, and non-invasively measuring the pressure pulse wave using a pressure sensor.

The body movement measurement unit 21 includes a tri-axis acceleration sensor, and measures the movement of the user's body (body movement) using this sensor. The body movement measurement unit 21 may include a circuit that converts the format of an output from the tri-axis acceleration sensor into a format that is readable to the control unit 23.

The environment measurement unit 22 measures environmental information that may affect mental and physical conditions of the user (in particular the blood pressure). The environment measurement unit 22 may include, for example, an atmospheric temperature sensor, a humidity sensor, an illuminance sensor, an altitude sensor, a position sensor, and so on. The environment measurement unit 22 may include a circuit that converts the format of outputs from these sensors and so on into a format that is readable to the control unit 23.

The control unit 23 performs various kinds of processing, such as controlling each unit of the biological information analysis system 10, acquiring data from the measurement unit 2, storing the acquired data in the recording unit 27, processing and analyzing data, and inputting and outputting data. The control unit 23 includes a hardware processor (hereinafter referred to as the "CPU") a ROM (Read Only Memory), a RAM (Random Access Memory), and so on. Processing that is performed by the control unit 23, which will be described later, is realized by the CPU reading and executing a program stored in the ROM or the storage unit 27. The RAM functions as a work memory that is used by the control unit 23 when performing various kinds of processing. Although acquisition of data from the measurement unit 2 and the storing of data in the storage unit 27 are performed by the control unit 23 in the present embodiment, it is possible to employ a configuration in which the measurement unit 2 directly stores (writes) data in the storage unit 27.

Each of the constituent components of the embodiment such as a measurement unit, an indicator extraction unit, a processing unit, a determination unit, a risk database, an input unit, an output unit, a case database, and so on may be implemented as pieces of hardware in the biological information analysis system 10. The indicator extraction unit, the processing unit, and the determination unit may receive an executable program stored in the storage unit 27, and execute the program. The indicator extraction unit, the processing unit, and the determination unit may receive data from the blood pressure measurement unit 20, the body movement measurement unit 21, the environment measurement unit 22, the input unit 24, the output unit 25, the communication unit 26, the storage unit 27, and so on as required. Databases such as the risk database and the case database may be implemented using the storage unit 27 and so on, and store pieces of information that are arranged such that a data search and data accumulation can be easily performed. Here, for example, the configuration, operations, and so on of the biological information analysis system 10 are disclosed in JP 2016-082069A. The contents of this disclosure are incorporated herein by reference. Also, the configuration, operations, and so on of the blood pressure measurement unit are disclosed in JP 2016-087003A. The contents of this disclosure are incorporated herein by reference.

The input unit 24 provides an operation interface for the user. For example, an operation button, a switch, a touch panel, and so on may be used.

The output unit 25 provides an interface that outputs information to the user. For example, a display device (such as a liquid crystal display) that outputs information using images, an audio output device or a beeper that outputs information using audio, an LED that outputs information by blinking, a vibration device that outputs information by vibrating, and so on may be used.

The communication unit 26 performs data communication with another device. Any data communication method such as a wireless LAN or Bluetooth (registered trademark) may be used.

The storage unit 27 is a storage medium that can store data and from which data can be read out, and stores programs that are to be executed by the control unit 23, pieces of measurement data acquired from the measurement units, and various kinds of data acquired by processing the pieces of measurement data, and so on. The storage unit 27 is a medium that accumulates pieces of information that are to be stored, through an electrical, magnetic, optical, mechanical, or chemical action. For example, a flash memory is used. The storage unit 27 may be a portable unit such as a memory card, or built into the biological information analysis system 10.

At least one unit or all units out of the body movement measurement unit 21, environment measurement unit 22, the control unit 23, the input unit 24, the output unit 25, and the storage unit 27 may be configured as a device that is separate from the main body 11. That is, as long as the blood pressure measurement unit 20 and the main body 11 that incorporates a circuit that controls the blood pressure measurement unit 20 are configured to be wearable on a wrist, the configurations of other units can be freely designed. If this is the case, the main body 11 cooperates with another unit via the communication unit 26. Various configurations can be conceived of. For example, the functions of the control unit 23, the input unit 24, and the output unit 25 may be realized using a smartphone application, and required data may be acquired from an activity monitor that has the functions of the body movement measurement unit 21 and the environment measurement unit 22. Also, a sensor that measures biological information other than blood pressure may be provided. For example, a sleep sensor, a pulse oximeter (an SpO2 sensor), a respiration sensor (a flow sensor), a blood-sugar level sensor, and the like may be combined.

Although the sensor (the blood pressure measurement unit 20) that measures blood pressure and the component (including the control unit 23 and so on) that performs processing to analyze blood pressure waveform data are provided in one device in the present embodiment, they may be provided in separate members. In the present embodiment, the component (including the control unit 23 and so on) that performs processing to analyze biological information is referred to as a biological information analysis device, and the device that includes the combination of the measurement unit and the biological information analysis device is referred to as a biological information analysis system. However, these names are given for descriptive purposes, and the measurement unit and the component that performs processing to analyze biological information may be referred to as a biological information analysis device as a whole, or other names may be used.

Measurement of Blood Pressure Waveform

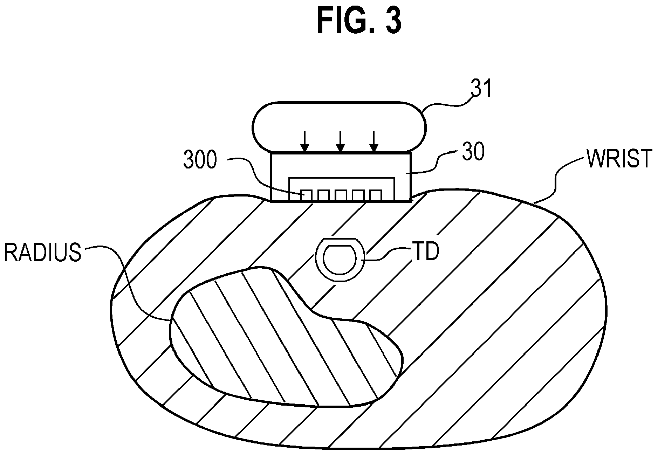

FIG. 3 is a cross-sectional view schematically showing the configuration of the blood pressure measurement unit 20 and a state in which measurement is performed. The blood pressure measurement unit 20 includes a pressure sensor 30 and a pressurizing mechanism 31 for pressing the pressure sensor 30 against a wrist. The pressure sensor 30 includes a plurality of pressure detection elements 300. The pressure detection elements 300 detect pressure and convert the pressure into an electrical signal. For example, elements that utilize a piezoresistive effect may be preferably used. The pressurizing mechanism 31 includes, for example, an air bag and a pump that adjusts the internal pressure of the air bag. As a result of the control unit 23 controlling the pump to increase the internal pressure of the air bag, the air bag expands and the pressure sensor 30 is pressed against the surface of the skin. Note that the pressurizing mechanism 31 may be any mechanism as long as it can adjust the pressing force of the pressure sensor 30 applied to the surface of the skin, and is not limited to a mechanism that uses an air bag.

Upon the biological information analysis system 10 being worn on a wrist and activated, the control unit 23 controls the pressurizing mechanism 31 of the blood pressure measurement unit 20 to keep the pressing force of the pressure sensor 30 in an appropriate state (a tonometry state). Then, pressure signals detected by the pressure sensor 30 are sequentially acquired by the control unit 23. Pressure signals acquired from the pressure sensor 30 are generated by digitizing analogue physical amounts (e.g. voltage values) output by the pressure detection elements 300, through an A/D converter circuit or the like that employs a well-known technology. Preferable analogue values such as current values or resistance values may be employed as the analogue physical amounts, depending on the type of the pressure detection elements 300. Signal processing such as the aforementioned A/D conversion may be performed using a predetermined circuit provided in the blood pressure measurement unit 20, or performed by another unit (not shown) provided between the blood pressure measurement unit 20 and the control unit 23. Each pressure signal acquired by the control unit 23 corresponds to an instantaneous value of the internal pressure of the radial artery TD. Therefore, it is possible to acquire time-series data regarding blood pressure waveforms by acquiring pressure signals with time granularity and continuity that make it possible to ascertain a blood pressure waveform for each heartbeat. The control unit 23 stores the pressure signals sequentially acquired from the pressure sensor 30, in the storage unit 27, together with information regarding points in time at which the pressure signals were measured. The control unit 23 may store the acquired pressure signals in the storage unit 27 without change, or store the pressure signals in the storage unit 27 after performing required signal processing on the pressure signals. Required signal processing includes, for example, processing that is performed to calibrate each pressure signal such that the amplitude of the pressure signal matches the blood pressure value (e.g. the brachial blood pressure), processing that is performed to reduce or remove noise in each pressure signal, and so on.

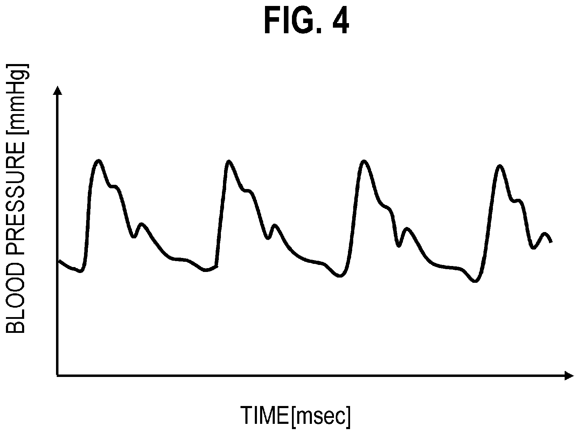

FIG. 4 shows a blood pressure waveform measured by the blood pressure measurement unit 20. The horizontal axis indicates time and the vertical axis indicates blood pressure. Although the sampling frequency may be set to any value, it is preferably set to be no less than 100 Hz so that characteristics of the shape of a waveform corresponding to one heartbeat can be reproduced. Typically, the period of one heartbeat is approximately one second, and therefore approximately one hundred or more data points can be acquired on a waveform corresponding to one heartbeat.

The blood pressure measurement unit 20 according to the present embodiment is advantageous in terms of the following.

The blood pressure measurement unit 20 can measure a blood pressure waveform for each heartbeat. As a result, it is possible to acquire various indicators related to blood pressure, the state of the heart, cardiovascular risks, and so on, based on the characteristics of the shape of the blood pressure waveform. In addition, it is possible to monitor for instantaneous values of blood pressure. Therefore, it is possible to instantaneously detect a blood pressure surge (a sudden rise in the blood pressure value), and to detect changes in blood pressure and irregularities in a blood pressure waveform that may occur in a very short period of time (corresponding to one to several heartbeats) without missing them.

As a portable blood pressure meter, a blood pressure meter that is to be worn on a wrist or an upper arm and employs an oscillometric method to measure blood pressure has come into practical use. However, a conventional portable blood pressure meter can only measure the mean value of blood pressure based on changes in the internal pressure of a cuff during a period of several seconds to a dozen or so seconds corresponding to a plurality of heartbeats, and cannot acquire time-series data regarding a blood pressure waveform for each heartbeat, unlike the blood pressure measurement unit 20 according to the present embodiment.

The blood pressure measurement unit 20 can record time-series data regarding blood pressure waveforms. By acquiring time-series data regarding blood pressure waveforms, and, for example, discerning characteristics of the blood pressure waveform related to temporal changes, or performing a frequency analysis on the time-series data to extract a specific frequency component, it is possible to acquire various indicators related to blood pressure, the state of the heart, cardiovascular risks, and so on.

The device employs a portable (wearable) type configuration, and less burden is placed on the user during measurement. Therefore, continuous measurement for a long time, and even 24-hour blood pressure monitoring, can be relatively easily performed. Also, since the device is of a portable type, changes in not only blood pressure under resting conditions, but also an ambulatory blood pressure (for example, during daily life or exercise) can be measured. As a result, it is possible to grasp how blood pressure is affected by behaviours in daily life (such as sleeping, eating, commuting, working, and taking medicine) and exercise, for example.

Conventional products are types of devices that measure blood pressure under resting conditions, with an arm or a wrist fixed to a blood pressure measurement unit, and cannot measure changes in blood pressure in daily life or during exercise, unlike the biological information analysis system 10 according to the present embodiment.

The blood pressure measurement unit 20 can be easily combined or linked with other sensors. For example, it is possible to make an evaluation of a cause-effect relationship or a composite evaluation with information that can be acquired by other sensors (e.g. body movement, environmental information such as an atmospheric temperature, biological information such as SpO2 and respiration information).

Biological Information Analysis Device

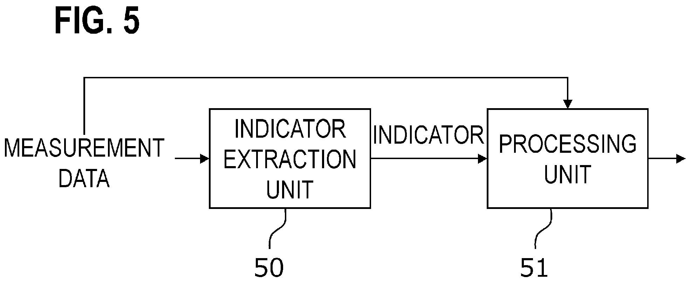

FIG. 5 is a block diagram illustrating processing that is performed by the biological information analysis device 1. As shown in FIG. 5, the biological information analysis device 1 includes an indicator extraction unit 50 and a processing unit 51. In the present embodiment, processing performed by the indicator extraction unit 50 and the processing unit 51 may be realized by the control unit 23 executing a program that is required for the processing. The program may be stored in the storage unit 27. When the control unit 23 executes the required program, the subject program stored in the ROM or storage unit 27 is loaded to the RAM. Then, the control unit 23 interprets and executes the program loaded to the RAM, using the CPU, to control each constituent component. Note that at least one or all of the processing procedures executed by the indicator extraction unit 50 and the processing unit 51 may be realized using a circuit such as an ASIC or an FPGA. Alternatively, at least one or all of the processing procedures executed by the indicator extraction unit 50 and the processing unit 51 may be realized using a computer (e.g. a smartphone, a tablet terminal, a personal computer, or a cloud server) that is separate from the main body 11.

The indicator extraction unit 50 acquires time-series data regarding blood pressure waveforms, which have been consecutively measured by the blood pressure measurement unit 20, from the storage unit 27. The indicator extraction unit 50 extracts, from the acquired time-series data regarding blood pressure waveforms, indicators that are related to characteristics of the blood pressure waveforms. Here, characteristics of a blood pressure waveform include, for example, characteristics of the shape of a blood pressure waveform corresponding to one heartbeat, temporal changes in a blood pressure waveform, and frequency components of a blood pressure waveform. However, characteristics of a blood pressure waveform are not limited to those listed above. The extracted indicators are output to the processing unit 51. There are various characteristics and indicators regarding a blood pressure waveform, and the characteristics and indicators that are to be extracted may be designed or selected as appropriate according to the purpose of processing that is to be performed by the processing unit 51. Characteristics and indicators that can be extracted from measurement data regarding blood pressure waveforms according to the present embodiment will be described later in detail.

When obtaining indicators, the indicator extraction unit 50 may use measurement data that has been acquired by the body movement measurement unit 21 and/or measurement data that has been acquired by the environment measurement unit 22, in addition to measurement data regarding blood pressure waveforms. Also, although not shown in the drawings, pieces of measurement data that have been acquired by a sleep sensor, an SpO2 sensor, a respiration sensor (a flow sensor), a blood-sugar level sensor, and the like may be combined with one another. By performing complex analysis on a plurality of kinds of measurement data acquired by a plurality of sensors, it is possible to perform more advanced information analysis of a blood pressure waveform. For example, it is possible to classify pieces of data regarding blood pressure waveforms according to states of the user, such as a resting state and a moving state, a state when an atmospheric temperature is high and a state when it is low, a light sleep state and a deep sleep state, a breathing state and an apnea state, and so on. Alternatively, it is possible to extract information regarding the influence of body movement, an activity amount, activity intensity, a change in an atmospheric temperature, apnea, the user's breathing, etc. on blood pressure, and thus evaluate the cause-effect relationship, the correlation, etc. between pieces of measurement data.

The processing unit 51 receives the indicators extracted by the indicator extraction unit 50. The processing unit 51 performs processing that is based on the received indicators. Various kinds of processing can be conceived of as processing that is based on the indicators. For example, the processing unit 51 may provide the values of the extracted indicators or changes in the values to a user, a doctor, a public health nurse, or the like to prompt the utilization of the indicators in the fields of health care, treatment, health guidance, and so on. Alternatively, the processing unit 51 may estimate cardiovascular risks from the extracted indicators, or provide guidelines for health maintenance or risk mitigation. Furthermore, when an increase in the risk of a cardiac disease occurring is detected or predicted based on an indicator, the processing unit 51 may inform the user or his/her doctor, or perform control to prevent the user from performing an action that burdens his/her heart and so on, or to prevent a cardiovascular event from occurring.

Information Acquired from Blood Pressure Waveform

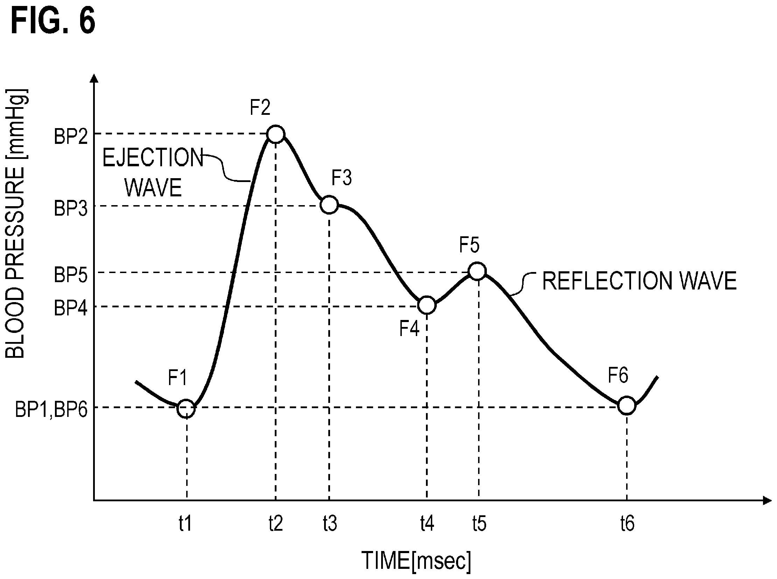

FIG. 6 shows a waveform (a blood pressure waveform) of a pressure pulse wave from a radial artery corresponding to one heartbeat. The horizontal axis indicates time t (msec) and the vertical axis indicates blood pressure BP (mmHg).

A blood pressure waveform is the waveform of a composite wave constituted by an "ejection wave" that is generated when the heart contracts and pumps out blood, and a "reflection wave" that is generated when an ejection wave is reflected at a branch point of a peripheral vessel or an artery. The following shows examples of characteristic points that can be extracted from a blood pressure waveform corresponding to one heartbeat.

- A point F1 is the rising point of the pressure pulse wave. The point F1 corresponds to the ejection start point of the heart, i.e. the point at which the aortic valve opens.
- A point F2 is a point at which the amplitude (the pressure) of the ejection wave is at the maximum (a first peak).
- A point F3 is an inflection point that appears midway in a drop in the ejection wave, due to a reflection wave being superimposed.
- A point F4 is the minimum point, which appears between the ejection wave and the reflection wave, and is also referred to as a notch. This point corresponds to the point at which the aortic valve closes.
- A point F5 is the peak of the reflection wave (a second peak), which appears after the point F4.
- A point F6 is the end point of one heartbeat, and corresponds to the ejection start point of the next heartbeat, i.e. the start point of the next heartbeat.

The indicator extraction unit 50 may use any algorithm to detect the above-described characteristic points. For example, the indicator extraction unit 50 may perform computations to obtain an nth order differential waveform of a blood pressure waveform, and detect the zero-crossing points to extract the characteristic points (the inflection points) of the blood pressure waveform (the points F1, F2, F4, F5, and F6 can be detected from the first order differential waveform, and the point F3 can be detected from the second order differential waveform or the fourth order differential waveform). Alternatively, the indicator extraction unit 50 may read out, from the storage unit 27, a waveform pattern on which the characteristic points have been arranged in advance, and perform fitting of the waveform pattern to the target blood pressure waveform to specify the respective positions of the characteristic points.

The indicator extraction unit 50 performs computations based on time t and pressure BP of each of the above-described characteristic points F1 to F6, and can thus obtain various kinds of information (values, characteristic amounts, indicators, etc.) from the blood pressure waveform of one heartbeat. The following are typical examples of information that can be acquired from a blood pressure waveform. Note that tx and BPx respectively represent time and blood pressure corresponding to a characteristic point Fx.

- Pulse Wave Interval (Period of Heartbeat) TA = t6 - t1
- Heart Rate PR = 1/TA
- Pulse Wave Rising Time UT = t2 - t1
- Systole TS = t4 - t1
- Diastole TD = t6 - t4
- Reflection Wave Delay Time = t3 - t1
- Maximum Blood Pressure (Systolic Blood Pressure) SBP = BP2
- Minimum Blood Pressure (Diastolic Blood Pressure) DBP = BP1
- Mean Blood Pressure MAP = (Area of Blood Pressure Waveform from t1 to t6)/Period of Heartbeat TA
- Mean Blood Pressure during Systole = (Area of Blood Pressure Waveform from t1 to t4)/Systole TS
- Mean Blood Pressure during Diastole = (Area of Blood Pressure Waveform from t4 to t6)/Diastole TD
- Pulse Pressure PP = Maximum Blood Pressure SBP - Minimum Blood Pressure DBP
- Late Systolic Pressure SBP2 = BP3
- AI (Augmentation Index) = (Late Systolic Pressure SBP2 - Minimum Blood Pressure DBP)/Pulse Pressure PP

Basic statistics of these pieces of information (values, characteristic amounts, and indicators) can also be used as indicators. Basic statistics include, for example, representative values (a mean value, a median value, a mode value, the maximum value, the minimum value, and so on) and the degree of scatter (dispersion, a standard deviation, a coefficient of variation, and so on). Temporal changes in these pieces of information (values, characteristic values, and indicators) can also be used as indicators.

In addition, the indicator extraction unit 50 can also acquire an indicator called BRS (Baroreflex Sensitivity) by performing computations on pieces of heartbeat information. This indicator indicates the ability to regulate blood pressure to be constant. Examples of methods for calculating the indicator include a spontaneous sequence method. This is a method for only extracting a sequence in which the maximum blood pressure SBP and the pulse wave interval TA consecutively rise or fall over the period of three or more heartbeats in synchronization with each other, plotting the maximum blood pressure SBP and the pulse wave interval TA onto a two-dimensional plane, and defining the inclination of the regression line obtained through a least squares method as the BRS.

As described above, the use of the biological information analysis system 10 according to the present embodiment makes it is possible to acquire various kinds of information from blood pressure waveform data. However, the biological information analysis system 10 need not implement all of the functions that are required to acquire all of the kinds of information described above. The biological information analysis system 10 need only implement functions that are required to acquire necessary information, depending on the configuration of the biological information analysis system 10, who the user is, the purpose of use, the location of use, and so on. Also, each function may be provided as a program module (a piece of application software), and the biological information analysis system 10 may employ a mechanism with which a function can be added by installing a necessary program module on the biological information analysis system 10.

The following illustrates an example, which is a specific application, of the biological information analysis system 10.

Example 1

The present example relates to a method for evaluating and visualizing an influence of exercise on a user's cardiac function, based on time-series data regarding blood pressure waveforms.

Although it is known that exercise has the effect of improving blood pressure, it is difficult for the user to imagine how much of an effect exercise has on the user. Also, although too much exercise can actually be harmful to the user's body, it is difficult for the user to know how much of a load is placed on his/her heart and so on due to the exercise that he/she is performing.

During an experiment on the subject, the inventors of the present invention observed changes in blood pressure waveforms before and after exercise, and ascertained the following phenomena. (1) Exercise causes a temporal change in a blood pressure waveform, and upon the exercise being complete, the blood pressure waveform gradually returns to its original state. (2) The amount of change in a blood pressure waveform and the amount of time required to return to a pre-exercise blood pressure waveform (a returning speed) depend on the activity amount of exercise performed. (3) The amount of change in a blood pressure waveform, and the amount of time required to return to a pre-exercise blood pressure waveform (a returning speed) also depend on the user's cardiovascular characteristics. Based on such findings, the present example proposes an algorithm for evaluating the degree of influence of exercise on cardiac function based on time-series data regarding blood pressure waveforms.

A biological information analysis device 1 according to the present example uses characteristic amounts extracted from a blood pressure waveform for each heartbeat, to evaluate changes in blood pressure waveforms before and after exercise, and estimate cardiovascular characteristics. Examples of characteristic amounts of a blood pressure waveform that can be used include the AI (Augmentation Index), the systolic blood pressure SBP, the diastole blood pressure DBP, the mean blood pressure MAP, the heart rate PR, a time difference DTp between the systolic blood pressure SBP and the late systolic pressure SBP2 (t3 - t2 in FIG. 6), and so on. AI, SBP, DBP, and MAP among these characteristic amounts temporarily decrease as a result of exercise, and then gradually increase and return to their respective original values. In contrast, PR and DTp temporarily increase as a result of exercise, and then gradually decrease and return to their respective original states. Note that it is not possible to grasp such changes in the characteristic amounts unless an ambulatory blood pressure waveform is accurately monitored for each heartbeat. In other words, it is not possible to grasp such changes in the characteristics amounts from blood pressure data obtained by a blood pressure meter that employs an oscillometric method and cannot measure blood pressure waveforms, or blood pressure data measured in a resting state.

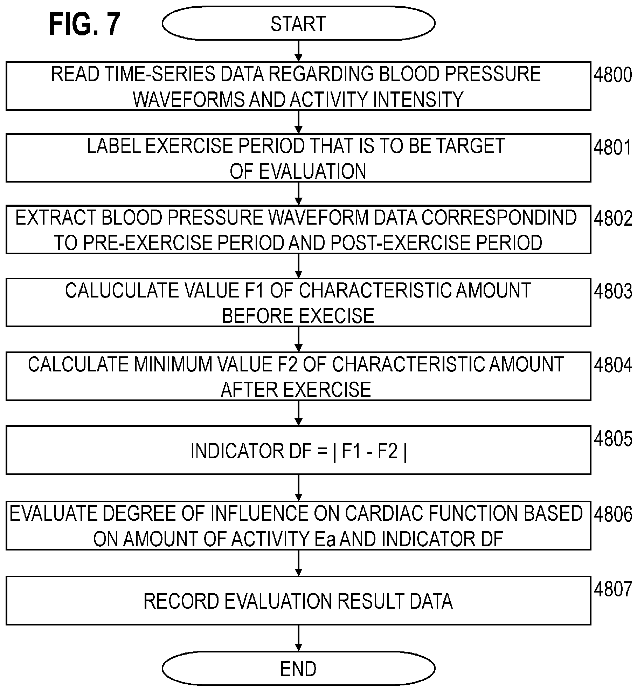

The following describes processing according to the present example with reference to the flowchart shown in FIG. 7. Here, it is presumed that pieces of time-series data regarding blood pressure waveforms measured by the blood pressure measurement unit 20 and pieces of time-series data regarding activity intensity (METs) measured by the body movement measurement unit 21 are accumulated in the storage unit 27 or another data storage (which may be a cloud sever).

The indicator extraction unit 50 reads time-series data regarding blood pressure waveforms and time-series data regarding activity intensity from the storage unit 27 or another data storage (step 4800). Next, the indicator extraction unit 50 detects periods of time during which exercise has been performed, from the time-series data regarding activity intensity, and labels an exercise period that is to be the target of evaluation (step 4801). For example, a period of time that satisfies the following conditions (1) to (3) is labeled as the target of evaluation.

(1) Exercise (a state where activity intensity is greater than 1 METs) continues for a time Tth1 or longer. Not only METs but also another indicator that indicates activity intensity (e.g. METs × body weight) may be used to represent activity intensity.

(2) The amount of activity (an Ex amount) throughout an entire period of time is no less than a threshold value Eth. Here, an Ex amount (exercise amount) is a value obtained by multiplying activity intensity (METs) by an activity time.

(3) Non-exercise periods (e.g. periods of time during which a state where activity intensity is no greater than 1 METs continues) that have a duration no less than a time Tth2 are respectively present before and after the period of time.

Note that the condition (2) may be determined based on, for example, another indicator related to exercise (e.g. statistics (a mean value, the maximum value, a mode value, and so on) of the intensity of exercise performed during the period of time) or calories consumed, instead of the activity amount. The threshold value Tth1 is set to be a time that is at least longer than the minimum unit time for which activity intensity can be measured. Also, the threshold value Tth2 is set to be of a duration whereby exercise performed in other periods of time does not influence evaluation. The threshold values Tth1, Tth2, and Eth used for the above-described determination processing are stored in the storage unit 27 in advance, for example.

In the following description, an exercise period labelled as the target of evaluation is referred to as a "target period", and the amount of activity in the target period is denoted as Ea, and the duration (length) of activity is denoted as Ta.

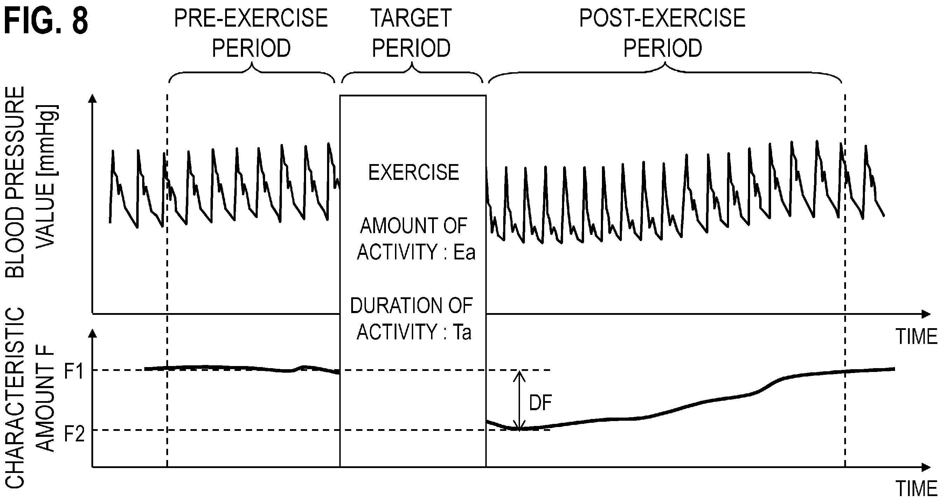

Next, the indicator extraction unit 50 acquires, from time-series data regarding blood pressure waveforms, blood pressure waveform data corresponding to a non-exercise period that is immediately before the target period (referred to as a "pre-exercise period") and blood pressure waveform data corresponding to a non-exercise period that is immediately after the target period (referred to as a "post-exercise period") (step 4802). The length of the pre-exercise period and the post-exercise period can be freely set (so as to be no greater than Tth2 at the maximum). FIG. 8 shows changes in blood pressure waveforms and values of the characteristic amount F of the blood pressure waveforms within a pre-exercise period, a target period (an exercise period), and a post-exercise period. FIG. 8 shows an example in which an AI value is used as the characteristic amount F of the blood pressure waveforms.

The indicator extraction unit 50 calculates a value F1, which is a characteristic amount F before exercise, based on blood pressure waveform data regarding the pre-exercise period (step 4803). For example, the value F1 is a mean value, a mode value, a median value, or the like of the characteristic amount F of the pre-exercise period. The indicator extraction unit 50 also calculates a minimum value F2 of the characteristic amount F in the post-exercise period, based on blood pressure waveform data regarding the post-exercise period (step 4804). Then, the indicator extraction unit 50 calculates an amount of change DF (= |F1-F2|) in characteristic amount (step 4805). The indicator DF represents how much a blood pressure waveform has changed as a result of exercise performed in the target period, and can be used as an indicator that represents the influence of exercise on the user's cardiac function. Note that, in the case of a characteristic amount that increases as a result of exercise (e.g. PR or DTp), a maximum value F2 of the characteristic amount F in the post-exercise period is to be calculated in step 4804.

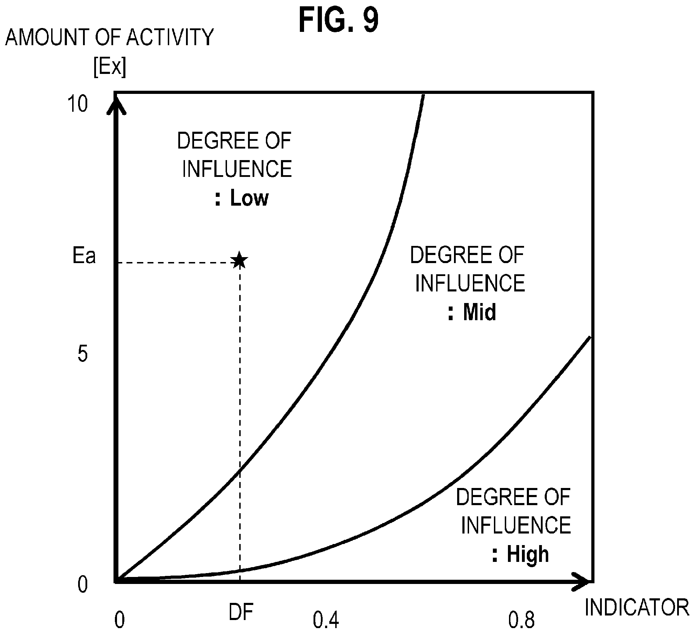

Subsequently, the processing unit 51 evaluates the degree of influence of the exercise on the cardiac function, based on the amount of activity Ea in the target period and the indicator DF (step S4806). In the present example, one of the values Low (degree of influence: small), Mid (degree of influence: middle), and High (degree of influence: large) is output as a result of evaluation R. For example, if the indicator DF is small (the amount of change in the characteristic amount F is small) despite the amount of activity Ea being large, an evaluation is made indicating that the cardiac function is less susceptible to the exercise. FIG. 9 is an example of a chart for determining the result of evaluation R, which is used by the processing unit 51. The horizontal axis indicates the value of the indicator DF, and the vertical axis indicates the amount of activity (Ex). Note that criteria for evaluation and the determination chart are merely examples, and another method may be employed. Also, for example, soft clustering or evaluation using continuous values may be employed instead of three-level evaluation.

The processing unit 51 generates evaluation result data in which, for example, pieces of information regarding the target period (the start time of exercise, the amount of activity Ea, and the duration of activity Ta), pieces of information regarding the pre-exercise period and the post-exercise period (the characteristic amounts F1 and F2), the indicator (DF) indicating the degree of influence, and the result of evaluation (R) are associated with each other, and stores the evaluation result data in the storage unit 27 (step 4807). If a plurality of target periods have been extracted in step 4800, the processing performed in steps 4802 to 4807 is repeated for each target period.

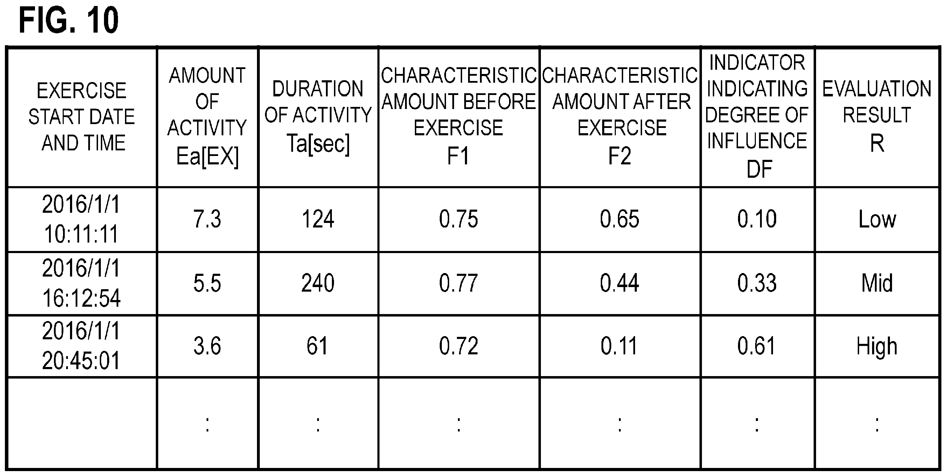

FIG. 10 shows examples of pieces of evaluation result data accumulated in the storage unit 27. As a result of such pieces of evaluation result data being accumulated, it is possible to grasp to what extent exercise influences the user's cardiac function, in an objective manner. Consequently, for example, the user can grasp how responsive his/her current cardiac function is to exercise, and can imagine the effect of exercise therapy, which will motivate the user to perform exercise regularly. In addition, the user can be aware of exercise methods that are dangerous to them, and can select a type of exercise therapy that is suitable for the user's cardiovascular characteristics and is not excessive. Also, doctors and public health nurses can utilize such data to instruct the user to perform appropriate exercise therapy that is suited to the user's cardiovascular characteristics.

Modifications

In addition to the amount of change DF in the characteristic amount F, the following indicators may be used as an indicator indicating the influence of exercise on cardiac function.

- The amount of time T required for the characteristic amount F that has changed as a result of exercise, to return to the state thereof in the pre-exercise period.
- A speed V at which the characteristic amount F that has changed as a result of exercise returns to the state thereof in the pre-exercise period.

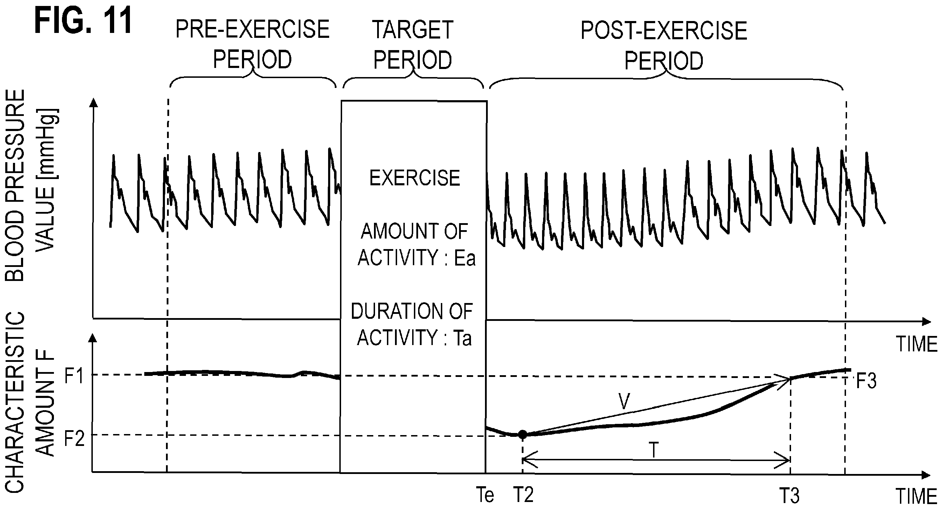

Both the indicators T and V indicate the resilience of the user's cardiac function. FIG. 11 shows examples of the indicators T and V in a case where AI values are used as values of the characteristic amount F of a blood pressure waveform. The indicators T and V are respectively calculated as follows.

$$T = T3 - T2$$

$$V = |F3 - F2|/|T3 - T2|$$

Here, T2 denotes a point in time at which the characteristic amount F is at the minimum in the post-exercise period, and F2 denotes the value of the characteristic amount at the point in time T2. T3 denotes a point in time in the post-exercise period, at which the characteristic amount F returns to the value F1, which is the value before exercise, and F3 denotes the value of the characteristic amount at the point in time T3. Note that a certain margin may be provided in determining whether or not the characteristic amount F has been restored. For example, it is possible to determine that the characteristic amount F "has been restored" if the difference or ratio between the value F1 of the characteristic amount before exercise and the value F3 of the characteristic amount after exercise is within a predetermined range. Also, when the end time of the target period (the point in time at which exercise ends) is denoted as Te, the indicators T and V may be obtained as follows:

$$T = T3 - Te$$

$$V = |F3 - Fe|/|T3 - Te|$$

where Fe denotes the value of the characteristic amount at the point in time Te.

As with the indicator DF, it is also possible to evaluate the degree of influence of exercise on cardiac function by using the indicators T and V. When evaluating the degree of influence, it is possible to use a plurality of indicators. For example, although a two-axis chart defined by the amount of activity Ea and the indicator DF is used in FIG. 9, it is possible to determine the degree of influence by using a three-axis chart defined by the amount of activity Ea, the indicator DF, and the indicator T, or by using a four-axis chart defined by the amount of activity Ea, the indicator DF, the indicator T, and the indicator V. Note that a combination and the number of indicators used for determination may be freely selected.

Example of Output

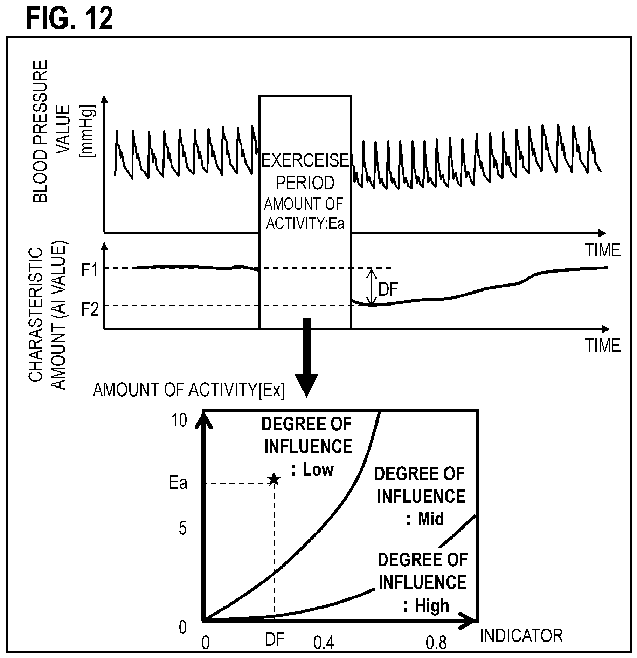

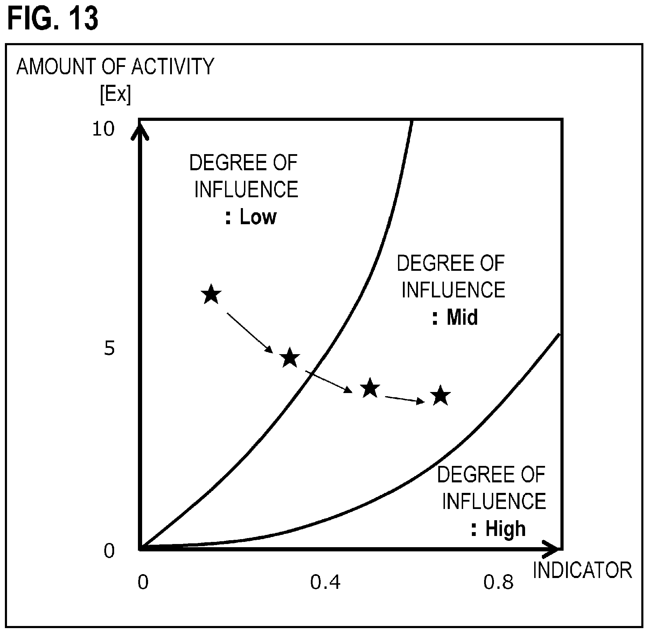

The processing unit 51 may output information obtained through the above-described evaluation processing to the output unit 25 or an external display device or the like (not shown). FIG. 12 shows an example of an information output screen output by the processing unit 51. The upper part shows a graph of information regarding the exercise period (the amount of activity Ea) and the blood pressure waveforms and the characteristic amount (the AI value) in the pre-exercise period and the post-exercise period, and the lower part shows a chart showing the result of evaluation (the star mark) of the exercise period. By looking at such a screen, the user can easily grasp the degree of influence of the exercise that he/she has performed, on his/her cardiac function. FIG. 13 shows another example of an information output screen output by the processing unit 51. The results of evaluation (the star marks) of a plurality of exercises (four times in the example shown in FIG. 13) are displayed on the chart in chronological order. By looking at such a screen, the user can intuitively grasp changes in the degree of influence of the exercises that he/she has performed, on his/her cardiac function.

The configurations according to the above-described embodiment and examples are no more than specific examples of configurations according to the present invention, and are not intended to limit the scope of the present invention. The present invention may employ various specific configurations without departing from the technical idea thereof.

The technical idea disclosed in the present description can be specified as the following aspects of the present invention.

Supplementary Note 1

A biological information analysis device comprising:

a hardware processor; and a memory that is configured to store a program, wherein the hardware processor is configured to execute the program to

acquire, from time-series data regarding blood pressure waveforms consecutively measured by a sensor that is configured to be worn on a body part of a user and to be capable of non-invasively measuring a blood pressure waveform for each heartbeat, data regarding a blood pressure waveform in a pre-exercise period that is a period of time before the user performs exercise, and data regarding a blood pressure waveform in a post-exercise period that is a period of time after the user performs the exercise; extract an indicator indicating the influence of the exercise on the user's cardiac function, based on characteristics of the blood pressure waveform in the pre-exercise period and the characteristics of the blood pressure waveform in the post-exercise period; and

perform processing that is based on the indicator thus extracted.

Supplementary Note 2

A biological information analysis system comprising:

a sensor that is configured to be worn on a body part of a user and to be capable of non-invasively measuring a blood pressure waveform for each heartbeat; a hardware processor; and a memory that is configured to store a program, wherein the hardware processor is configured to execute the program to

acquire, from time-series data regarding blood pressure waveforms consecutively measured by a sensor that is configured to be worn on a body part of a user and to be capable of non-invasively measuring a blood pressure waveform for each heartbeat, data regarding a blood pressure waveform in a pre-exercise period that is a period of time before the user performs exercise, and data regarding a blood pressure waveform in a post-exercise period that is a period of time after the user performs the exercise; extract an indicator indicating the influence of the exercise on the user's cardiac function, based on characteristics of the blood pressure waveform in the pre-exercise period and the characteristics of the blood pressure waveform in the post-exercise period; and

perform processing that is based on the indicator thus extracted.

Supplementary Note 3

A biological information analysis method comprising:

a step of acquiring, from time-series data regarding blood pressure waveforms consecutively measured by a sensor that is configured to be worn on a body part of a user and to be capable of non-invasively measuring a blood pressure waveform for each heartbeat, data regarding a blood pressure waveform in a pre-exercise period that is a period of time before the user performs exercise, and data regarding a blood pressure waveform in a post-exercise period that is a period of time after the user performs the exercise, using at least one hardware processor;
a step of extracting an indicator indicating the influence of the exercise on the user's cardiac function, based on characteristics of the blood pressure waveform in the pre-exercise period and the characteristics of the blood pressure waveform in the post-exercise period, using at least one hardware processor; and
a step of performing processing that is based on the indicator thus extracted, using at least one hardware processor.

INDEX TO THE REFERENCE NUMERALS

1 ... biological information analysis device, 2 ... measurement unit 10 ... biological information analysis system, 11 ... main body, 12 ... belt 20 ... blood pressure measurement unit, 21 ... body movement measurement unit, 22 ... environment measurement unit, 23 ... control unit, 24 ... input unit, 25 ... output unit, 26 ... communication unit, 27 ... storage unit 30 ... pressure sensor, 31 ... pressurizing mechanism, 300 ... pressure detection element
50 ... indicator extraction unit, 51 ... processing unit

**Claims**

1.  A biological information analysis device comprising:

    an indicator extraction unit configured to acquire, from time-series data regarding blood pressure waveforms consecutively measured by a sensor that is configured to be worn on a body part of a user and to be capable of non-invasively measuring a blood pressure waveform for each heartbeat, data regarding a blood pressure waveform in a pre-exercise period that is a period of time before the user performs exercise, and data regarding a blood pressure waveform in a post-exercise period that is a period of time after the user performs the exercise, and extract an indicator indicating the influence of the exercise on the user's cardiac function, based on characteristics of a blood pressure waveform in the pre-exercise period and characteristics of a blood pressure waveform in the post-exercise period; and
    a processing unit configured to perform processing that is based on the indicator thus extracted.

2.  The biological information analysis device according to claim 1,
    wherein the indicator extraction unit is configured to calculate the indicator based on a difference between the characteristics of the blood pressure waveform in the pre-exercise period and the characteristics of the blood pressure waveform in the post-exercise period.

3.  The biological information analysis device according to claim 1,
    wherein the indicator extraction unit is configured to calculate the indicator based on an amount of time required for the characteristics of the blood pressure waveform, which has changed as a result of the exercise, to return to a state thereof in the pre-exercise period.

4.  The biological information analysis device according to claim 1,
    wherein the indicator extraction unit is configured to calculate the indicator based on a speed at which the characteristics of the blood pressure waveform, which has changed as a result of the exercise, return to a state thereof in the pre-exercise period.

5.  The biological information analysis device according to any one of claims 1 to 4,
    wherein the indicator extraction unit is configured to use, as characteristics of blood pressure waveforms, at least one of an AI (Augmentation Index), a systolic blood pressure, a diastolic blood pressure, a mean blood pressure, a heart rate, and a difference in time between a systolic blood pressure and a late systolic pressure.

6.  The biological information analysis device according to any one of claims 1 to 5,
    wherein the processing unit is configured to evaluate the degree of influence of the exercise on cardiac function, based on the indicator and an indicator that is related to the intensity and/or the duration of the exercise.

7.  The biological information analysis device according to claim 6,
    wherein the processing unit is configured to perform processing to output information regarding the degree of influence.

8.  A biological information analysis system comprising:

    a sensor that is configured to be worn on a body part of a user and to be capable of non-invasively measuring a blood pressure waveform for each heartbeat; and
    the biological information analysis device according to any one of claims 1 to 7, the biological information analysis device being configured to analyze biological information, using data regarding blood pressure waveforms consecutively measured by the sensor.

9. A program that causes a processor to function as the indicator extraction unit and the processing unit of the biological information analysis device according to any one of claims 1 to 7.

10. A biological information analysis method comprising:

a step of acquiring, from time-series data regarding blood pressure waveforms consecutively measured by a sensor that is configured to be worn on a body part of a user and to be capable of non-invasively measuring a blood pressure waveform for each heartbeat, data regarding a blood pressure waveform in a pre-exercise period that is a period of time before the user performs exercise, and data regarding a blood pressure waveform in a post-exercise period that is a period of time after the user performs the exercise, and extracting an indicator indicating the influence of the exercise on the user's cardiac function, based on characteristics of the blood pressure waveform in the pre-exercise period and characteristics of the blood pressure waveform in the post-exercise period; and
a step of performing processing that is based on the indicator thus extracted.

# FIG. 1

10

TD

11

12

**FIG. 2**

# FIG. 3

EP 3 427 654 A1

# FIG. 4

# FIG. 5

MEASUREMENT DATA → INDICATOR EXTRACTION UNIT (50) → INDICATOR → PROCESSING UNIT (51) →

EP 3 427 654 A1

FIG. 6

EP 3 427 654 A1

**FIG. 7**

```
                    ┌──────────────────────┐
                    │        START         │
                    └──────────────────────┘
                               │
                               ▼
┌──────────────────────────────────────────────────────┐  ┌──────┐
│  READ TIME-SERIES DATA REGARDING BLOOD PRESSURE        │  │ 4800 │
│  WAVEFORMS AND ACTIVITY INTENSITY                      │  └──────┘
└──────────────────────────────────────────────────────┘
                               │
                               ▼
┌──────────────────────────────────────────────────────┐  ┌──────┐
│  LABEL EXERCISE PERIOD THAT IS TO BE TARGET            │  │ 4801 │
│  OF EVALUATION                                         │  └──────┘
└──────────────────────────────────────────────────────┘
                               │
                               ▼
┌──────────────────────────────────────────────────────┐  ┌──────┐
│  EXTRACT BLOOD PRESSURE WAVEFORM DATA CORRESPONDIND    │  │ 4802 │
│  TO PRE-EXERCISE PERIOD AND POST-EXERCISE PERIOD       │  └──────┘
└──────────────────────────────────────────────────────┘
                               │
                               ▼
┌──────────────────────────────────────────────────────┐  ┌──────┐
│  CALUCULATE VALUE F1 OF CHARACTERISTIC AMOUNT          │  │ 4803 │
│  BEFORE EXECISE                                        │  └──────┘
└──────────────────────────────────────────────────────┘
                               │
                               ▼
┌──────────────────────────────────────────────────────┐  ┌──────┐
│  CALCULATE MINIMUM VALUE F2 OF CHARACTERISTIC AMOUNT   │  │ 4804 │
│  AFTER EXERCISE                                        │  └──────┘
└──────────────────────────────────────────────────────┘
                               │
                               ▼
┌──────────────────────────────────────────────────────┐  ┌──────┐
│  INDICATOR DF = | F1 - F2 |                            │  │ 4805 │
└──────────────────────────────────────────────────────┘  └──────┘
                               │
                               ▼
┌──────────────────────────────────────────────────────┐  ┌──────┐
│  EVALUATE DEGREE OF INFLUENCE ON CARDIAC FUNCTION BASED│  │ 4806 │
│  ON AMOUNT OF ACTIVITY Ea AND INDICATOR DF             │  └──────┘
└──────────────────────────────────────────────────────┘
                               │
                               ▼
┌──────────────────────────────────────────────────────┐  ┌──────┐
│  RECORD EVALUATION RESULT DATA                         │  │ 4807 │
└──────────────────────────────────────────────────────┘  └──────┘
                               │
                               ▼
                    ┌──────────────────────┐
                    │         END          │
                    └──────────────────────┘
```

# FIG. 8

PRE-EXERCISE PERIOD | TARGET PERIOD | POST-EXERCISE PERIOD

BLOOD PRESSURE VALUE [mmHg]

EXERCISE

AMOUNT OF ACTIVITY : Ea

DURATION OF ACTIVITY : Ta

TIME

CHARACTERISTIC AMOUNT F

F1

F2

DF

TIME

# FIG. 9

# FIG. 10

| EXERCISE START DATE AND TIME | AMOUNT OF ACTIVITY Ea[EX] | DURATION OF ACTIVITY Ta[sec] | CHARACTERISTIC AMOUNT BEFORE EXERCISE F1 | CHARACTERISTIC AMOUNT AFTER EXERCISE F2 | INDICATOR INDICATING DEGREE OF INFLUENCE DF | EVALUATION RESULT R |
|---|---|---|---|---|---|---|
| 2016/1/1 10:11:11 | 7.3 | 124 | 0.75 | 0.65 | 0.10 | Low |
| 2016/1/1 16:12:54 | 5.5 | 240 | 0.77 | 0.44 | 0.33 | Mid |
| 2016/1/1 20:45:01 | 3.6 | 61 | 0.72 | 0.11 | 0.61 | High |
| | : | : | : | : | : | : |

EP 3 427 654 A1

FIG. 11

## FIG. 12

## FIG. 13

EP 3 427 654 A1

## INTERNATIONAL SEARCH REPORT

| | International application No. |
|---|---|
| | PCT/JP2017/015284 |

A. CLASSIFICATION OF SUBJECT MATTER
*A61B5/02*(2006.01)i, *A61B5/022*(2006.01)i, *A61B5/029*(2006.01)i, *A61B5/08*
(2006.01)i

According to International Patent Classification (IPC) or to both national classification and IPC

B. FIELDS SEARCHED

Minimum documentation searched (classification system followed by classification symbols)
A61B5/02, A61B5/022, A61B5/029, A61B5/08

Documentation searched other than minimum documentation to the extent that such documents are included in the fields searched
| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 8-229012 A (Colin Corp.), | 1-5,7-10 |
| Y | 10 September 1996 (10.09.1996), paragraphs [0015] to [0051]; fig. 4 to 10 (Family: none) | 6 |
| X | JP 8-229011 A (Colin Corp.), | 1-5,7-10 |
| Y | 10 September 1996 (10.09.1996), paragraphs [0016] to [0046]; fig. 3 to 9 (Family: none) | 6 |
| Y | JP 2004-121865 A (Seiko Epson Corp.), 22 April 2004 (22.04.2004), paragraphs [0067] to [0080]; fig. 8 & US 5941837 A columns 20 to 23 & EP 809965 A1 | 6 |

| ☒ Further documents are listed in the continuation of Box C. | ☐ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 13 June 2017 (13.06.17) | 27 June 2017 (27.06.17) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2017/015284 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 2003-24310 A (Seiko Epson Corp.), 08 December 2003 (08.12.2003), entire text; all drawings (Family: none) | 1-10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008061824 A
- JP 2005532111 A
- JP 2016082069 A
- JP 2016087003 A